# EUROPEAN PATENT SPECIFICATION

(11) **EP 4 364 771 B1**
(45) Date of publication and mention of the grant of the patent: **30.07.2025**
(21) Application number: 23203471.0
(22) Date of filing: 13.10.2023
(51) Int. Cl.: A61M 16/08, A61M 16/04, A61B 5/097, A61B 5/08, A61M 16/10, A61B 5/087, A61M 16/00

(54) **SENSING AND SECRETION BYPASS APPARATUSES AND ASSOCIATED METHODS**
SENSOR- UND SEKRETIONSBYPASS-VORRICHTUNGEN UND ZUGEHÖRIGE VERFAHREN
APPAREILS DE DÉRIVATION DE DÉTECTION ET DE SÉCRÉTION ET PROCÉDÉS ASSOCIÉS

(30) Priority: 04.11.2022 US 202218052666
(43) Date of publication of application: 08.05.2024
(73) Proprietor: Honeywell International Inc., Charlotte, NC 28202 (US)
(72) Inventor: AVASTHI, Rahul, Charlotte, NC 28202 (US); BENTLEY, Ian, Charlotte, NC 28202 (US)
(74) Representative: Haseltine Lake Kempner LLP

(56) References cited:
- US-A1- 2017 266 399
- US-A1- 2018 078 723
- US-A1- 2021 379 317
- US-A1- 2022 023 558

## Description

### TECHNOLOGICAL FIELD

Embodiments of the present disclosure relate generally to sensing and secretion bypass apparatuses for measuring air flow and associated methods.

### BACKGROUND

US2021/379317A1 discloses a valve including a body having an internal chamber, first and second body ports in fluid communication with the chamber with the first port configured for fluid communication with a patient connection and the second body port configured for fluid communication with a ventilator, a body passageway in fluid communication with the chamber and with ambient air exterior of the body, and a check valve seal positioned to seal the body passageway to permit the flow of gas within the chamber through the body passageway to the exterior of the body and to prevent the flow of ambient air exterior of the body through the body passageway into the chamber. In alternative embodiments, the valve is incorporated into the patient connection or constructed as a separate part connectable to the patient connection. US2018/078723A1 discloses a secretion removal assembly configured to connect to a respiratory secretion retention device, the respiratory secretion retention device adapted to fluidly connect to an artificial airway, the secretion removal assembly including a connector adapted to connect to a port of the respiratory secretion retention device; and a bag in fluid communication with the connector can be provided. In another aspect of this embodiment, the bag is adapted to collect the secretions that emit from the port of the respiratory secretion retention device. In yet another aspect of this embodiment, the secretion removal assembly further can include a tube having a first end and a second end opposite the first end, the first end in fluid communication with the connector and the second end in fluid communication with the bag.
US2017/266399A1 discloses a flow sensor system for ventilation treatment comprising a flow conduit configured to allow gas flow between a first region and a second region, the flow conduit defining a lumen for the gas flow; a flow restrictor disposed within the lumen of the flow conduit between the first region and the second region; a first absolute pressure sensor disposed adjacent to the first region of the flow conduit and configured to measure a pressure of the gas flow at the first region of the flow conduit; and a second absolute pressure sensor disposed adjacent to the second region of the flow conduit and configured to measure pressure of the gas flow at the second region of the flow conduit.
Applicant has identified many technical challenges and difficulties associated with sensing and secretion bypass apparatuses for measuring air flow and associated methods. Through applied effort, ingenuity, and innovation, Applicant has solved problems related to sensing and secretion bypass apparatuses for measuring air flow and associated methods by developing solutions embodied in the present disclosure, which are described in detail below.

### BRIEF SUMMARY

Various embodiments described herein relate to sensing and secretion bypass apparatuses for measuring air flow and associated methods.

In accordance with the invention, a sensing and secretion bypass apparatus is provided. The sensing and secretion bypass apparatus includes an air flow sensor. The sensing and secretion bypass apparatus includes an adapter comprising a chamber having a first end configured to receive an air flow and a second end connected to the air flow sensor. In this regard, the air flow comprises secretions. The sensing and secretion bypass apparatus includes an elevated member disposed within the chamber proximate the second end and configured to prevent the secretions from entering the air flow sensor. The sensing and secretion bypass apparatus includes a disposal housing in fluid communication with the chamber of the adapter and configured to collect the secretions received via the first end of the chamber.

In some embodiments, the first end is configured to receive the secretions from an expiratory circuit or inspiratory circuit.

In some embodiments, the expiratory circuit or inspiratory circuit is associated with a mechanical ventilation device.

The disposal housing is in fluid communication with the chamber via a bottom side of the chamber such that the disposal housing is configured to collect the secretions by the force of gravity.

The elevated member comprises a first end. The elevated member is disposed within the chamber such that the first end of the elevated member is in contact with the bottom side of the chamber.

The elevated member comprises a second end. The elevated member is disposed within the chamber such that the second end of the elevated member is separated from the bottom side of the chamber by a first distance.

In some embodiments, the air flow sensor is configured to receive the air flow without the secretions from the second end of the chamber.

In some embodiments, the air flow sensor comprises an air flow measurement apparatus configured to measure one or more properties associated with the air flow.

In some embodiments, the one or more properties associated with the air flow comprise one or more of a velocity, a pressure, a humidity, a temperature, an oxygen concentration, a carbon dioxide concentration, a nitric oxide concentration, a methane concentration, a presence of a volatile organic compound, a mass, and a volume associated with the air flow.

In accordance with another aspect of the invention, a method of manufacturing a sensing and secretion bypass apparatus is provided. The method of manufacturing a sensing and secretion bypass apparatus includes providing an air flow sensor. The method of manufacturing a sensing and secretion bypass apparatus includes providing an adapter comprising a chamber having a first end configured to receive an air flow and a second end connected to the air flow sensor. In this regard, the air flow comprises secretions. The method of manufacturing a sensing and secretion bypass apparatus includes providing an elevated member disposed within the chamber proximate the second end and configured to prevent the secretions from entering the air flow sensor. The method of manufacturing a sensing and secretion bypass apparatus includes providing a disposal housing in fluid communication with the chamber of the adapter and configured to collect the secretions received via the first end of the chamber.

In some embodiments, the first end is configured to receive the secretions from an expiratory circuit or inspiratory circuit.

In some embodiments, the expiratory circuit or inspiratory circuit is associated with a mechanical ventilation device.

The disposal housing is in fluid communication with the chamber via a bottom side of the chamber such that the disposal housing is configured to collect the secretions by the force of gravity.

The elevated member comprises a first end. The elevated member is disposed within the chamber such that the first end of the elevated member is in contact with the bottom side of the chamber.

The elevated member comprises a second end.

The elevated member is disposed within the chamber such that the second end of the elevated member is separated from the bottom side of the chamber by a first distance.

In some embodiments, the air flow sensor is configured to receive the air flow without the secretions from the second end of the chamber.

In some embodiments, the air flow sensor comprises an air flow measurement apparatus configured to measure one or more properties associated with the air flow.

In some embodiments, the one or more properties associated with the air flow comprise one or more of a velocity, a pressure, a humidity, a temperature, an oxygen concentration, a carbon dioxide concentration, a nitric oxide concentration, a methane concentration, a presence of volatile organic compounds, a mass, and a volume associated with the air flow.

In accordance with one aspect of the disclosure, which is not part of the invention, another sensing and secretion bypass apparatus is provided. In some embodiments, the sensing and secretion bypass apparatus includes an upper portion connected to an expiratory circuit or inspiratory circuit. In this regard, the upper portion receives an air flow from the expiratory circuit or inspiratory circuit, wherein the air flow comprises secretions. In some embodiments, the sensing and secretion bypass apparatus includes a lower portion removably attached to the upper portion. In some embodiments, the sensing and secretion bypass apparatus includes a collection housing disposed on an inner surface of the lower portion. In this regard, the collection housing is configured to collect the secretions in the air flow. In some embodiments, the sensing and secretion bypass apparatus includes an air flow measurement apparatus disposed on an inner surface of the upper portion. In this regard, the air flow measurement apparatus is configured to measure one or more properties associated with the air flow when the lower portion is detached from the upper portion.

**In** some embodiments, the upper portion and the lower portion define a chamber when attached.

**In** some embodiments, the collection housing is configured to collect the secretions in the air flow prior to the air flow measurement apparatus measuring the one or more properties associated with the air flow.

**In** some embodiments, the one or more properties associated with the air flow comprise one or more of a velocity, a pressure, a humidity, a temperature, an oxygen concentration, a carbon dioxide concentration, a nitric oxide concentration, a methane concentration, a presence of a volatile organic compound, a mass, and a volume.

**In** some embodiments, the air flow measurement apparatus is configured to measure the one or more properties associated with the air flow when the lower portion is attached to the upper portion.

In some embodiments, the expiratory circuit or inspiratory circuit is associated with a mechanical ventilation device.

In some embodiments, the lower portion is removably attached to the upper portion via one or more clips.

In accordance with another aspect of the disclosure, which is not part of the invention, another method of manufacturing a sensing and secretion bypass apparatus is provided. In some embodiments, the method of manufacturing a sensing and secretion bypass apparatus includes providing an upper portion connected to an expiratory circuit or inspiratory circuit. In this regard, the upper portion receives an air flow from the expiratory circuit or inspiratory circuit, wherein the air flow comprises secretions. In some embodiments, the method of manufacturing a sensing and secretion bypass apparatus includes providing a lower portion removably attached to the upper portion. In some embodiments, the method of manufacturing a sensing and secretion bypass apparatus includes a collection housing disposed on an inner surface of the lower portion. In this regard, the collection housing is configured to collect the secretions in the air flow. In some embodiments, the method of manufacturing a sensing and secretion bypass apparatus includes providing an air flow measurement apparatus disposed on an inner surface of the upper portion. In this regard, the air flow measurement apparatus is configured to measure one or more properties associated with the air flow when the lower portion is detached from the upper portion.

In some embodiments, the upper portion and the lower portion define a chamber when attached.

In some embodiments, the collection housing is configured to collect the secretions in the air flow prior to the air flow measurement apparatus measuring the one or more properties associated with the air flow.

In some embodiments, the one or more properties associated with the air flow comprise one or more of a velocity, a pressure, a humidity, a temperature, an oxygen concentration, a carbon dioxide concentration, a nitric oxide concentration, a methane concentration, a presence of volatile organic compounds, a mass, and a volume.

In some embodiments, the air flow measurement apparatus is configured to measure the one or more properties associated with the air flow when the lower portion is attached to the upper portion.

In some embodiments, the expiratory circuit or inspiratory circuit is associated with a mechanical ventilation device.

In some embodiments, the lower portion is removably attached to the upper portion via one or more clips.

The above summary is provided merely for purposes of summarizing some example embodiments to provide a basic understanding of some aspects of the present disclosure. Accordingly, it will be appreciated that the above-described embodiments are merely examples and should not be construed to narrow the scope or spirit of the disclosure in any way. It will be appreciated that the scope of the present disclosure encompasses many potential embodiments in addition to those here summarized, some of which will be further described below.

### BRIEF DESCRIPTION OF THE DRAWINGS

Reference will now be made to the accompanying drawings. The components illustrated in the figures may or may not be present in certain embodiments described herein. Some embodiments may include fewer (or more) components than those shown in the figures in accordance with an example embodiment of the present disclosure.
FIG. 1 illustrates a perspective view of an example sensing and secretion bypass apparatus in accordance with one or more embodiments of the present invention;
FIG. 2 illustrates a cross-sectional view of the example sensing and secretion bypass apparatus in accordance with one or more embodiments of the present invention;
FIG. 3 illustrates an example user interface of a mechanical ventilation device associated with the example sensing and secretion bypass apparatus in accordance with one or more embodiments of the present disclosure;
FIG. 4 illustrates a flowchart of an example method of manufacturing the example sensing and secretion bypass apparatus in accordance with one or more embodiments of the present invention;
FIG. 5 illustrates a perspective view of an example sensing and secretion bypass apparatus in accordance with one or more (unclaimed) embodiments of the present disclosure;
FIG. 6 illustrates a cross-sectional view of the example sensing and secretion bypass apparatus in accordance with one or more (unclaimed) embodiments of the present disclosure;
FIG. 7 illustrates an example user interface of a mechanical ventilation device associated with the example sensing and secretion bypass apparatus in accordance with one or more embodiments of the present disclosure;
FIG. 8 illustrates a flowchart of an example method of manufacturing the example sensing and secretion bypass apparatus in accordance with one or more (unclaimed) embodiments of the present disclosure; and
FIG. 9 illustrates a block diagram of an example computer processing device in accordance with one or more embodiments of the present disclosure.

### DETAILED DESCRIPTION

Example embodiments will be described more fully hereinafter with reference to the accompanying drawings, in which some, but not all embodiments of disclosure are shown. Indeed, embodiments of the disclosure may be embodied in many different forms and should not be construed as limited to the embodiments set forth herein; rather, these embodiments are provided so that this disclosure will satisfy applicable legal requirements. Like numbers refer to like elements throughout.

### Overview

Example embodiments disclosed herein address technical problems associated with sensing and secretion bypass apparatuses for measuring air flow and associated methods. As would be understood by one skilled in the field to which this disclosure pertains, there are numerous example scenarios in which a user may use sensing and secretion bypass apparatuses for measuring air flow and associated methods.

In many applications, it is often necessary to measure the properties associated with an air flow. For example, in individuals who are undergoing mechanical ventilation it is necessary to measure the air flowing out of the individual's lungs (e.g., expiratory air flow) and/or the air flowing into an individual's lungs (e.g., inspiratory air flow) to monitor the individual's health and/or the effectiveness of the mechanical ventilation. For example, measuring the oxygen concentration of the air flowing out of the individual's lungs can be an indicator of the individual's health and/or the effectiveness of the mechanical ventilation.

Example solutions for measuring expiratory air flow and/or inspiratory air flow include, for example, placing an air flow sensor in series with a tube from which air is expelled from an individual's lungs in mechanical ventilation and/or a tube from which air is provided to an individual's lungs in mechanical ventilation (e.g., an expiratory tube and/or inspiratory tube proximal to the patient) and measuring the expiratory air flow and/or inspiratory air flow as it moves through the air flow sensor. However, in some examples, there may be secretions (e.g., condensed water vapor, mucus, fluid, and/or organic material present in an individual's respiratory and/or digestive system) present in the expiratory air flow and/or inspiratory air flow. The secretions present in the expiratory air flow and/or inspiratory air flow may cause the air flow sensor to clog (e.g., secretions, such as mucus, may stick to the air flow sensor), measurements taken by the air flow sensor to be inaccurate (e.g., secretions in the air flow may alter the properties of the air flow), and/or the air flow sensor to fail. This can harm the individual's health and trigger alarms causing medical staff to respond, disrupting the workflow of medical staff. In many implementations, the only way to ensure measurements taken by the air flow sensor are accurate and prevent the air flow sensor from clogging, is to perform frequent inspections and regular cleaning of the air flow sensor and/or regularly replace the air flow sensor with a new air flow sensor. However, regular inspection and cleaning of the air flow sensor and/or replacement of the air flow sensor results in less efficient utilization of resources (e.g., medical staff must spend time inspecting and cleaning and/or replacing the air flow sensor), may cause harm to an individual's health because it is often necessary to detach the air flow sensor from the expiratory tube and/or inspiratory tube in order to inspect and clean and/or replace the air flow sensor, and may expose medical staff to infections (e.g., the secretions may be a biohazard).

Thus, to address these and/or other issues related to measuring air flow, example systems, apparatuses, and/or methods for measuring air flow are disclosed herein. For example, an embodiment in this disclosure, described in greater detail below, includes an adapter with a chamber having a first end for receiving an air flow having secretions and a second end connected to an air flow sensor. An elevated member is disposed within the chamber proximate the second end that prevents the secretions from entering the air flow sensor. A disposal housing is in fluid communication with the chamber and configured to collect the secretions. As a result, in some examples, secretions are prevented from interfering with measurements made by the air flow sensor by at least the elevated member and the secretions can be collected and disposed of without interfering with the measurement of the air flow (e.g., the disposal housing may be removed for disposal of the secretions without halting the air flow measurements).

As another example, an embodiment in this disclosure, not part of the invention and described in greater detail below, includes an upper portion that may receive an air flow from an expiratory circuit and/or inspiratory circuit connected to the upper portion. In some examples, a lower portion may be removably attached to the upper portion. In some examples, a collection housing may be disposed on an inner surface of the lower portion and be configured to collect secretions in the air flow. In some examples, an air flow measurement apparatus may be configured to measure one or more properties associated with the air flow when the lower portion is detached from the upper portion. As a result, in some examples, the secretions are prevented from interfering with measurements made by the air flow sensor (e.g., the secretions collect in the collection housing) and the secretions can be collected and disposed of without interfering with the measurement of the air flow (e.g., the lower portion may be detached from the upper portion to dispose of the secretions while the air flow measurement apparatus continues to measure the one or more properties associated with the air flow).

### Example Sensing and Secretion Bypass Apparatus

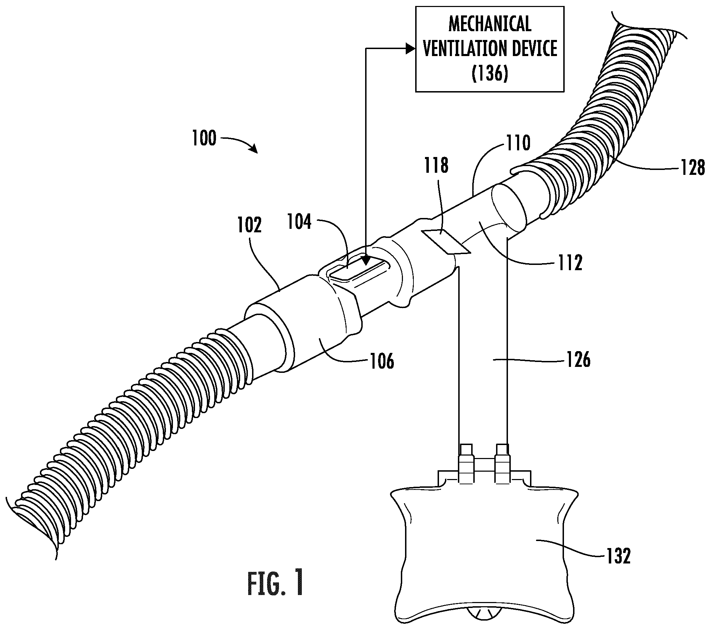

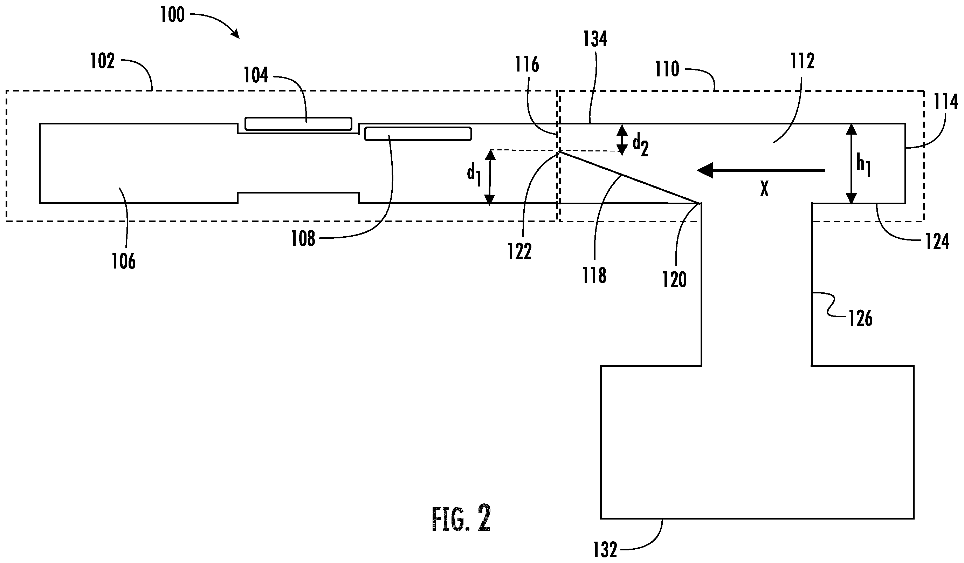

With reference to FIG. 1 and FIG. 2, embodiments herein provide for an example sensing and secretion bypass apparatus 100. The sensing and secretion bypass apparatus 100 is configured to measure an air flow and bypass secretions present in the air flow.

The sensing and secretion bypass apparatus 100 includes an air flow sensor 102. The air flow sensor 102 may include an air flow sensor chamber 106. The air flow sensor 102 may include an air flow measurement apparatus 108 disposed within the air flow sensor chamber 106. The air flow measurement apparatus 108 may be configured to measure one or more properties associated with the air flow. The one or more properties associated with the air flow may include one or more of a velocity, a pressure, a humidity, a temperature, an oxygen concentration or a presence of oxygen, a carbon dioxide concentration or a presence of carbon dioxide, a nitric oxide concentration or a presence of nitric oxide, a methane concentration or a presence of methane, a volatile organic compound concentration (e.g., benzene, ethylene glycol, formaldehyde, acetone, methylene chloride, tetrachloroethylene, toluene, xylene, and/or 1,3-butadiene) or a presence of a volatile organic compound, a mass, and a volume associated with the air flow. The air flow measurement apparatus 108 may be one or more of a hot-wire anemometer, thermopile, and/or differential pressure sensor or transducer.

Although the air flow sensor 102 depicted in FIGS. 1 and 2 includes a single air flow measurement apparatus 108, it would be understood by one skilled in the field to which this disclosure pertains that, in some embodiments, the air flow sensor 102 may include more than one air flow measurement apparatus 108. For example, the air flow sensor 102 may include multiple air flow measurement apparatuses 108 with each air flow measurement apparatus 108 configured to measure a particular property associated with the air flow (e.g., an air flow measurement apparatus 108 for measuring the velocity of the air flow, an air flow measurement apparatus 108 for measuring the humidity of the air flow, etc.).

In some embodiments, the air flow sensor 102 may include a controller 104. The controller 104 may be configured to receive one or more measurements made by the air flow measurement apparatus 108 from the air flow measurement apparatus 108. For example, the controller 104 may receive a velocity of the air flow. The controller 104 may receive the one or more measurements made by the air flow measurement apparatus 108 via a wired connection and/or a wireless connection (e.g., Bluetooth). In some embodiments, the controller 104 may be disposed on an outer surface of the air flow sensor 102. In other embodiments, the controller 104 may be disposed inside the air flow sensor chamber 106. Although the controller 104 and the air flow measurement apparatus 108 are depicted as separate components in FIGS. 1 and 2, it would be understood by one skilled in the field to which this disclosure pertains that, in some embodiments, the controller 104 and the air flow measurement apparatus 108 may be combined into a single component.

In some embodiments, the air flow sensor 102 may be comprised of any suitable material for facilitating the measurement of an air flow. In this regard, the air flow sensor 102 may comprise one or more of plastic, silicone, and/or metal (e.g., gold, copper, etc.). For example, if the air flow measurement apparatus 108 is a thermopile, the air flow sensor 102 may comprise plastic and/or metal (e.g., copper). As another example, if the air flow measurement apparatus 108 is a differential pressure sensor, the air flow sensor 102 may comprise plastic. In some embodiments, the air flow sensor 102 may be any suitable shape for facilitating the measurement of an air flow. For example, the air flow sensor 102 may be one or more of cylindrical, spherical, circular, rectangular, or cubical.

The sensing and secretion bypass apparatus 100 includes an adapter 110. The adapter 110 includes an adapter chamber 112. The adapter chamber 112 includes a first end 114 and a second end 116. The first end 114 of the adapter chamber 112 is configured to receive the air flow. In some embodiments, secretions may be present in the air flow. The secretions may include, for example, condensed water vapor, mucus, fluid, and/or organic material present in an individual's respiratory and/or digestive system. The second end 116 of the adapter chamber 112 may be connected to the air flow sensor 102.

An elevated member 118 is disposed within the adapter chamber 112. The elevated member 118 is disposed proximate the second end 116 of the adapter chamber 112. In this regard, the elevated member 118 includes a first end 120 and a second end 122. The first end 120 of the elevated member 118 is in contact with a bottom side 124 of the adapter chamber 112. The second end 122 of the adapter chamber 112 is separated from the bottom side 124 of the adapter chamber 112 by a first distance d₁.

The first distance d₁ is less than a height h₁ of the adapter chamber 112. As a result, there may be a second distance d₂ between the second end 122 of the elevated member 118 and a top side 134 of the adapter chamber 112 (e.g., d₁ plus d₂ are equal to hi). Said differently, the elevated member 118 is disposed within the adapter chamber 112 such that the elevated member 118 is proximate the second end 116 of the adapter chamber 112 and the elevated member 118 has a downward slope from the second end 116 of the adapter chamber 112 towards the first end 114 of the adapter chamber 112.

In some embodiments, after the air flow is received via the first end 114 of the adapter chamber 112, the air flow may flow through the adapter chamber 112 in direction x towards the second end 116 of the adapter chamber 112. As the air flow travels through the adapter chamber 112, the secretions present in the air flow may slowly begin to sink towards the bottom side 124 of the adapter chamber 112 due to the force of gravity. As a result, as the air flow moves through the adapter chamber 112, at least some of the secretions eventually impact the elevated member 118 (e.g., some of the secretions may sink to the bottom side 124 of the adapter chamber 112 due to the force of gravity before reaching the elevated member 118). After secretions impact the elevated member 118, the air flow (e.g., without the secretions) may continue to the second end 116 of the adapter chamber 112 and flow into the air flow sensor 102 (e.g., through the space defined by the second distance d₂ between the second end 122 of the elevated member 118 and a top side 134 of the adapter chamber 112). Accordingly, the elevated member 118 is configured to prevent secretions present in the air flow from entering the air flow sensor 102 while still enabling the air flow without the secretions to flow from the adapter chamber 112 into the air flow sensor 102 via the second end 116 of the adapter chamber 112.

In some embodiments, the adapter 110 may comprise any suitable material for receiving the air flow and being connected to the air flow sensor 102. Similarly, the elevated member 118 may be any suitable material for preventing secretions present in the air flow from entering the air flow sensor 102 while still enabling the air flow without the secretions to flow from the adapter chamber 112 into the air flow sensor 102 via the second end 116 of the adapter chamber 112. For example, the adapter 110 and/or the elevated member 118 may comprise one or more of plastic, silicone, and/or metal (e.g., gold, copper, etc.). In some embodiments, the adapter 110 may be any suitable shape for receiving the air flow and being connected to the air flow sensor 102. For example, the adapter 110 may be one or more of cylindrical, spherical, circular, rectangular, or cubical.

Although the adapter 110 and the air flow sensor 102 are depicted as separate components in FIGS. 1 and 2, it would be understood by one skilled in the field to which this disclosure pertains that, in some embodiments, the adapter 110 and the air flow sensor 102 may be combined into a single component.

The sensing and secretion bypass apparatus 100 includes a disposal housing 132. The disposal housing 132 is in fluid communication with the adapter chamber 112 of the adapter 110. For example, the disposal housing 132 is in fluid communication with the adapter chamber 112 via a tube 126. The disposal housing 132 is configured to collect secretions received from the adapter chamber 112. In this regard, as secretions sink to the bottom side 124 of the adapter chamber 112 and may fall into the disposal housing 132 via the tube 126 and be collected in the disposal housing 132. Additionally or alternatively, after secretions impact the elevated member 118 disposed in the adapter chamber 112, the secretions may begin to slide down the elevated member 118 due to the force of gravity (e.g., because the elevated member 118 is sloped). Accordingly, secretions that impact the elevated member 118 may eventually reach the first end 120 of the elevated member 118 and, at this point, may fall into the disposal housing 132 via the tube 126 and be collected in the disposal housing 132.

In some embodiments, after a certain quantity of secretions have collected in the disposal housing 132, the disposal housing 132 may be detached from the adapter chamber 112 to dispose of the secretions. The disposal housing 132 may be detached from the adapter chamber 112 while the air flow measurement apparatus 108 is measuring the air flow, thus, the secretions may be disposed of without interfering with the measurements made by the air flow measurement apparatus 108.

In some embodiments, the disposal housing 132 may comprise any suitable material for collecting the secretions from the adapter chamber 112. For example, the disposal housing 132 may comprise one or more of plastic, silicone, and/or metal (e.g., gold, copper, etc.). In some embodiments, the disposal housing 132 may be any suitable shape for collecting the secretions from the adapter chamber 112. For example, the disposal housing 132 may be one or more of cylindrical, spherical, circular, rectangular, or cubical.

In some embodiments, the adapter chamber 112 of the adapter 110 may be configured to receive the air flow from an expiratory circuit or inspiratory circuit 128 via the first end 114 of the adapter chamber 112. In some embodiments, the expiratory circuit or inspiratory circuit 128 may be associated with a mechanical ventilation device 136. For example, the expiratory circuit or inspiratory circuit 128 may be configured to receive expiratory air flow or inspiratory air flow associated with the mechanical ventilation device 136.

In some embodiments, the controller 104 may be in communication with the mechanical ventilation device 136, such as via a wired connection and/or a wireless connection (e.g., Bluetooth). The controller 104 may be configured to transmit one or more measurements made by the air flow measurement apparatus 108 to the mechanical ventilation device 136 (e.g., the one or more properties associated with the air flow measured by the air flow measurement apparatus 108). Similarly, the mechanical ventilation device 136 may be configured to receive the one or more measurements made by the air flow measurement apparatus 108. Although the controller 104 and the mechanical ventilation device 136 are depicted as separate components in FIG. 1, it would be understood by one skilled in the field to which this disclosure pertains that, in some embodiments, the controller 104 and the mechanical ventilation device 136 may be a single component. For example, in some embodiments, the controller 104 may be embodied within the mechanical ventilation device 136.

In some embodiments, the mechanical ventilation device 136 may be configured to perform further analysis on the one or more measurements made by the air flow measurement apparatus 108 to determine one or more properties associated with the air flow. For example, the air flow measurement apparatus 108 (e.g., a hot-wire anemometer) may measure a temperature (e.g., a temperature change) and the mechanical ventilation device 136 may determine a velocity associated with the air flow based on the measured temperature.

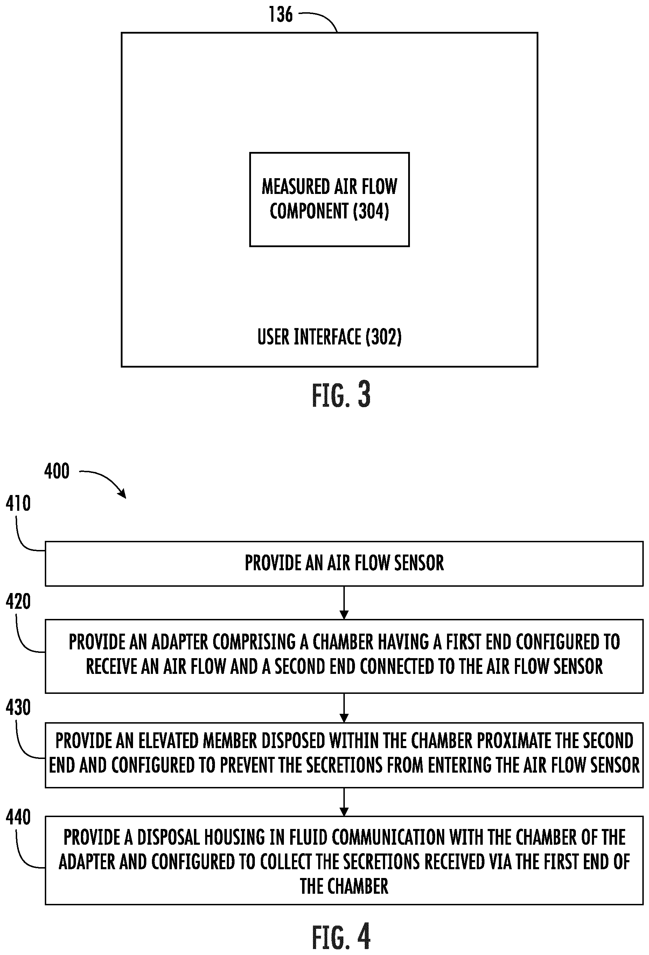

With reference to FIG. 3, in some embodiments, the mechanical ventilation device 136 may include a user interface 302. In some embodiments, the user interface 302 may include a measured air flow component 304. The measured air flow component 304 may be configured to display one or more measurements made by the air flow measurement apparatus 108 (e.g., the one or more measurements transmitted from the controller 104 and received by the mechanical ventilation device 136) and/or one or more properties associated with the air flow determined by the mechanical ventilation device 136 performing further analysis on the one or more measurements made by the air flow measurement apparatus 108. For example, the measured air flow component 304 may be configured to display the one or more properties associated with the air flow measured by the air flow measurement apparatus 108, including one or more of a velocity, a pressure, a humidity, a temperature, an oxygen concentration or a presence of oxygen, a carbon dioxide concentration or a presence of carbon dioxide, a nitric oxide concentration or a presence of nitric oxide, a methane concentration or a presence of methane, a volatile organic compound concentration (e.g., benzene, ethylene glycol, formaldehyde, acetone, methylene chloride, tetrachloroethylene, toluene, xylene, and/or 1,3-butadiene) or a presence of a volatile organic compound, a mass, and a volume associated with the air flow. In this regard, for example, the measured air flow component 304 may display text, symbols, graphs, and/or colors indicating one or more measurements made by the air flow measurement apparatus 108. For example, the measured air flow component 304 may display text indicating the velocity of the air flow.

Although described herein with respect to the mechanical ventilation device 136 having a user interface 302 with a measured air flow component 304 configured to display one or more measurements made by the air flow measurement apparatus 108, it would be understood by one skilled in the field to which this disclosure pertains that, in some embodiments, the controller 104 may include a user interface having a measured air flow component configured to display one or more measurements made by the air flow measurement apparatus 108.

### Example Method of Manufacturing a Sensing and Secretion Bypass Apparatus

Referring now to FIG. 4, a flowchart providing an example method 400 of manufacturing a sensing and secretion bypass apparatus is illustrated.

As shown in block 410, the method of manufacturing includes providing an air flow sensor. As described above, the air flow sensor may include an air flow sensor chamber. An air flow measurement apparatus may be disposed within the air flow sensor chamber. The air flow measurement apparatus may be configured to measure one or more properties associated with the air flow. The one or more properties associated with the air flow may include one or more of a velocity, a pressure, a humidity, a temperature, an oxygen concentration or a presence of oxygen, a carbon dioxide concentration or a presence of carbon dioxide, a nitric oxide concentration or a presence of nitric oxide, a methane concentration or a presence of methane, a volatile organic compound concentration (e.g., benzene, ethylene glycol, formaldehyde, acetone, methylene chloride, tetrachloroethylene, toluene, xylene, and/or 1,3-butadiene) or a presence of a volatile organic compound, a mass, and a volume associated with the air flow. As described above, the air flow measurement apparatus may be one or more of a hot-wire anemometer, thermopile, and/or differential pressure sensor or transducer.

As described above, the air flow sensor may include a controller. The controller may be configured to receive one or more measurements made by the air flow measurement apparatus from the air flow measurement apparatus. The controller may be in communication with a mechanical ventilation device, such as via a wired connection and/or a wireless connection (e.g., Bluetooth). The controller may be configured to transmit one or more measurements made by the air flow measurement apparatus to the mechanical ventilation device (e.g., the one or more properties associated with the air flow measured by the air flow measurement apparatus). Similarly, the mechanical ventilation device may be configured to receive the one or more measurements made by the air flow measurement apparatus. Although the controller and the mechanical ventilation device are depicted as separate components in FIG. 1, it would be understood by one skilled in the field to which this disclosure pertains that, in some embodiments, the controller and the mechanical ventilation device may be a single component. For example, in some embodiments, the controller may be embodied within the mechanical ventilation device.

As described above, in some embodiments, the mechanical ventilation device may include a user interface. In some embodiments, the user interface may include a measured air flow component. The measured air flow component may be configured to display one or more measurements made by the air flow measurement apparatus (e.g., the one or more measurements transmitted from the controller and received by the mechanical ventilation device). Additionally, as described above, although described herein with respect to the mechanical ventilation device having a user interface with a measured air flow component configured to display one or more measurements made by the air flow measurement apparatus, it would be understood by one skilled in the field to which this disclosure pertains that, in some embodiments, the controller may include a user interface having a measured air flow component configured to display one or more measurements made by the air flow measurement apparatus.

As shown in block 420, the method of manufacturing includes providing an adapter comprising an adapter chamber having a first end configured to receive an air flow and a second end connected to the air flow sensor. As described above the air flow includes secretions.

As shown in block 430, the method of manufacturing includes providing an elevated member disposed within the chamber proximate the second end and configured to prevent the secretions from entering the air flow sensor. As described above, after the air flow is received via the first end of the adapter chamber, the air flow may flow through the adapter chamber in direction x towards the second end of the adapter chamber. As the air flow travels through the adapter chamber, the secretions present in the air flow may slowly begin to sink towards the bottom side of the adapter chamber due to the force of gravity. As a result, as the air flow moves through the adapter chamber, at least some of the secretions eventually impact the elevated member (e.g., some of the secretions may sink to the bottom side of the adapter chamber due to the force of gravity before reaching the elevated member). After the secretions impact the elevated member, the air flow (e.g., without the secretions) may continue to the second end of the adapter chamber and flow into the air flow sensor (e.g., through the space defined by the second distance d₂ between the second end of the elevated member and a top side of the adapter chamber). Accordingly, the elevated member is configured to prevent secretions present in the air flow from entering the air flow sensor while still enabling the air flow without the secretions to flow from the adapter chamber into the air flow sensor via the second end of the adapter chamber.

As shown in block 440, the method of manufacturing includes providing a disposal housing in fluid communication with the chamber of the adapter and configured to collect the secretions received via the first end of the chamber. As described above, the disposal housing is in fluid communication with the adapter chamber of the adapter. For example, the disposal housing is in fluid communication with the adapter chamber via a tube. The disposal housing is configured to collect secretions received from the adapter chamber. In this regard, as secretions sink to the bottom side of the adapter chamber and may fall into the disposal housing via the tube and be collected in the disposal housing. Additionally or alternatively, after secretions impact the elevated member disposed in the adapter chamber, the secretions may begin to slide down the elevated member due to the force of gravity (e.g., because the elevated member is sloped). Accordingly, secretions that impact the elevated member may eventually reach the first end of the elevated member and, at this point, may fall into the disposal housing via the tube and be collected in the disposal housing.

### Example Sensing and Secretion Bypass Apparatus

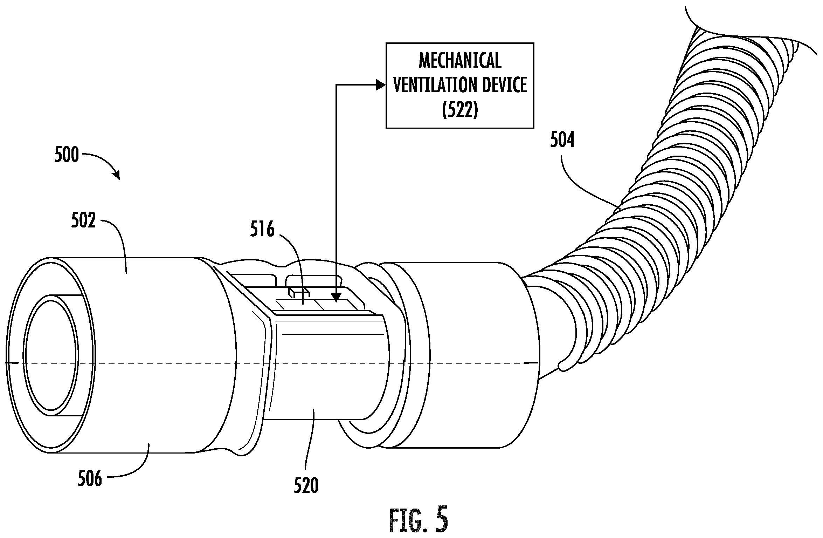

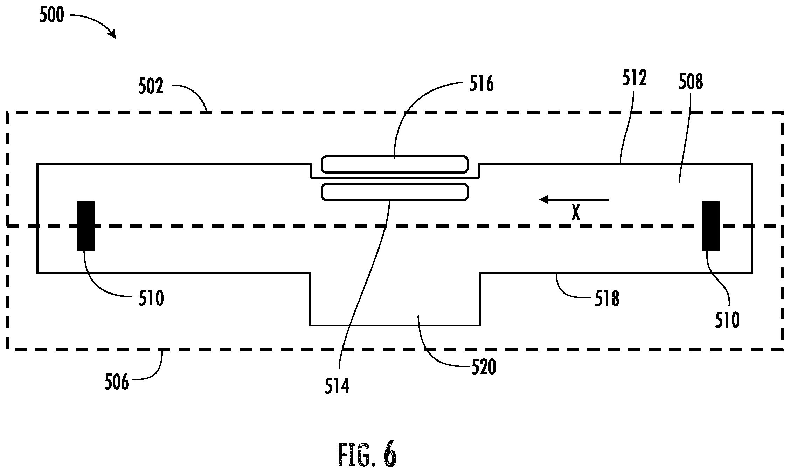

With reference to FIG. 5 and FIG. 6, embodiments herein, which are not part of the invention, provide for an example sensing and secretion bypass apparatus 500. In some embodiments, the sensing and secretion bypass apparatus 500 may be configured to measure an air flow and bypass secretions present in the air flow.

In some embodiments, the sensing and secretion bypass apparatus 500 may include an upper portion 502. The upper portion 502 may be connected to an expiratory circuit or inspiratory circuit 504. The upper portion 502 may receive an air flow from the expiratory circuit or inspiratory circuit 504. The expiratory circuit or inspiratory circuit 504 may be associated with a mechanical ventilation device 522. For example, the expiratory circuit or inspiratory circuit 504 may be configured to receive expiratory air flow or inspiratory air flow associated with the mechanical ventilation device 522.

In some embodiments, there may be secretions present in the air flow. The secretions may include, for example, condensed water vapor, mucus, fluid, and/or organic material present in an individual's respiratory and/or digestive system. Once the air flow has been received into the upper portion 502, the air flow may travel through the upper portion 502 in direction x.

In some embodiments, the sensing and secretion bypass apparatus 500 may include a lower portion 506. The lower portion 506 may be removably attached to the upper portion 502 of the sensing and secretion bypass apparatus 500. The upper portion 502 and the lower portion 506 may define a chamber 508 when attached (e.g., when the upper portion 502 and the lower portion 506 are attached). The lower portion 506 may be removably attached to the upper portion 502 via any means capable of making the lower portion 506 removably attached to the upper portion 502. For example, the lower portion 506 may be removably attached to the upper portion 502 via one or more clips 510.

In some embodiments, the upper portion 502 and/or the lower portion 506 may be comprised of any suitable material for facilitating the measurement of an air flow and bypassing secretions present in the air flow. For example, the upper portion 502 and/or the lower portion 506 may comprise one or more of plastic, silicone, and/or metal (e.g., gold, copper, etc.). In some embodiments, the upper portion 502 and/or the lower portion 506 may be any suitable shape for facilitating the measurement of an air flow and bypassing secretions present in the air flow. For example, the upper portion 502 and/or the lower portion 506 may be one or more of cylindrical, spherical, circular, rectangular, or cubical. In some embodiments, the upper portion 502 and the lower portion 506 may be the same shape.

In some embodiments, the sensing and secretion bypass apparatus 500 may include an air flow measurement apparatus 514 disposed on an inner surface 512 of the upper portion 502 (e.g., the air flow measurement apparatus 514 may be within the chamber 508 defined by the upper portion 502 and lower portion 506 when attached). The air flow measurement apparatus 514 may be configured to measure one or more properties associated with the air flow. The one or more properties associated with the air flow may include one or more of a velocity, a pressure, a humidity, a temperature, an oxygen concentration or a presence of oxygen, a carbon dioxide concentration or a presence of carbon dioxide, a nitric oxide concentration or a presence of nitric oxide, a methane concentration or a presence of methane, a volatile organic compound concentration (e.g., benzene, ethylene glycol, formaldehyde, acetone, methylene chloride, tetrachloroethylene, toluene, xylene, and/or 1,3-butadiene) or a presence of a volatile organic compound, a mass, and a volume associated with the air flow. The air flow measurement apparatus 514 may be one or more of a hot-wire anemometer, thermopile, and/or differential pressure sensor or transducer. In some embodiments, the air flow measurement apparatus 514 may be any suitable material for facilitating the measurement of the one or more properties associated with the air flow. For example, the air flow measurement apparatus 514 is a thermopile, the air flow measurement apparatus 514 may comprise plastic and/or metal (e.g., copper). As another example, if the air flow measurement apparatus 514 is a differential pressure sensor, the air flow measurement apparatus 514 may comprise plastic.

Although the sensing and secretion bypass apparatus 500 depicted in FIGS. 5 and 6 includes a single air flow measurement apparatus 514, it would be understood by one skilled in the field to which this disclosure pertains that, in some embodiments, the sensing and secretion bypass apparatus 500 may include more than one air flow measurement apparatus 514. For example, the sensing and secretion bypass apparatus 500 may include multiple air flow measurement apparatuses 514 with each air flow measurement apparatus 514 configured to measure a particular property associated with the air flow (e.g., an air flow measurement apparatus 514 for measuring the velocity of the air flow, an air flow measurement apparatus 514 for measuring the humidity of the air flow, etc.).

In some embodiments, the sensing and secretion bypass apparatus 500 may include a controller 516. The controller 516 may be configured to receive one or more measurements made by the air flow measurement apparatus 514 from the air flow measurement apparatus 514. For example, the controller 516 may receive a velocity of the air flow. The controller 516 may receive the one or more measurements made by the air flow measurement apparatus 514 via a wired connection and/or a wireless connection (e.g., Bluetooth). In some embodiments, the controller 516 may be disposed on an outer surface of the sensing and secretion bypass apparatus 500. In other embodiments, the controller 516 may be disposed inside on the inner surface 512 of the upper portion 502 (e.g., the controller 516 may be within the chamber 508 defined by the upper portion 502 and the lower portion 506 when attached). Although the controller 516 and the air flow measurement apparatus 514 are depicted as separate components in FIGS. 5 and 6, it would be understood by one skilled in the field to which this disclosure pertains that, in some embodiments, the controller 516 and the air flow measurement apparatus 514 may be combined into a single component.

In some embodiments, the sensing and secretion bypass apparatus 500 may include a collection housing 520 disposed on an inner surface 518 of the lower portion 506. In this regard, the collection housing 520 may be in fluid communication with the chamber 508 when the upper portion 502 and the lower portion 506 are attached. The collection housing 520 may be configured to collect secretions present in the air flow. In this regard, as the air flow travels through the upper portion 502 in direction x, the secretions present in the air flow may slowly begin to sink due to the force of gravity. In some embodiments, when the lower portion 506 is attached to the upper portion 502, the secretions may collect in the collection housing 520 (e.g., as the secretions sink due to the force of gravity). Accordingly, the collection housing 520 may be configured to collect the secretions present in the air flow such that the secretions do not interfere with measurements made by the air flow measurement apparatus 514. In some embodiments, when the lower portion 506 is detached from the upper portion 502, the secretions that have collected in the collection housing 520 may be disposed.

In some embodiments, the air flow measurement apparatus 514 of the sensing and secretion bypass apparatus 500 may be configured to measure the one or more properties associated with the air flow when the lower portion 506 is attached to the upper portion 502. In some embodiments, the air flow measurement apparatus 514 may be configured to measure the one or more properties associated with the air flow when the lower portion 506 is detached from the upper portion 502. In other words, the air flow measurement apparatus 514 may be configured to measure the one or more properties associated with the air flow irrespective of whether the lower portion 506 is attached to the upper portion 502. Accordingly, the sensing and secretion bypass apparatus 500 may be configured to continuously provide measurements of the one or more properties associated with the air flow (e.g., measurements may be provided when the lower portion 506 is detached from the upper portion 502 because the secretions in the collection housing 520 are being disposed of).

In some embodiments, the controller 516 may be in communication with the mechanical ventilation device 522, such as via a wired connection and/or a wireless connection (e.g., Bluetooth). The controller 516 may be configured to transmit one or more measurements made by the air flow measurement apparatus 514 to the mechanical ventilation device 522 (e.g., the one or more properties associated with the air flow measured by the air flow measurement apparatus 514). Similarly, the mechanical ventilation device 522 may be configured to receive the one or more measurements made by the air flow measurement apparatus 514. Although the controller 516 and the mechanical ventilation device 522 are depicted as separate components in FIG. 5, it would be understood by one skilled in the field to which this disclosure pertains that, in some embodiments, the controller 516 and the mechanical ventilation device 522 may be a single component. For example, in some embodiments, the controller 516 may be embodied within the mechanical ventilation device 522.

In some embodiments, the mechanical ventilation device 522 may be configured to perform further analysis on the one or more measurements made by the air flow measurement apparatus to determine one or more properties associated with the air flow. For example, the air flow measurement apparatus 514 (e.g., a hot-wire anemometer) may measure a temperature (e.g., a temperature change) and the mechanical ventilation device 522 may determine a velocity associated with the air flow based on the measured temperature.

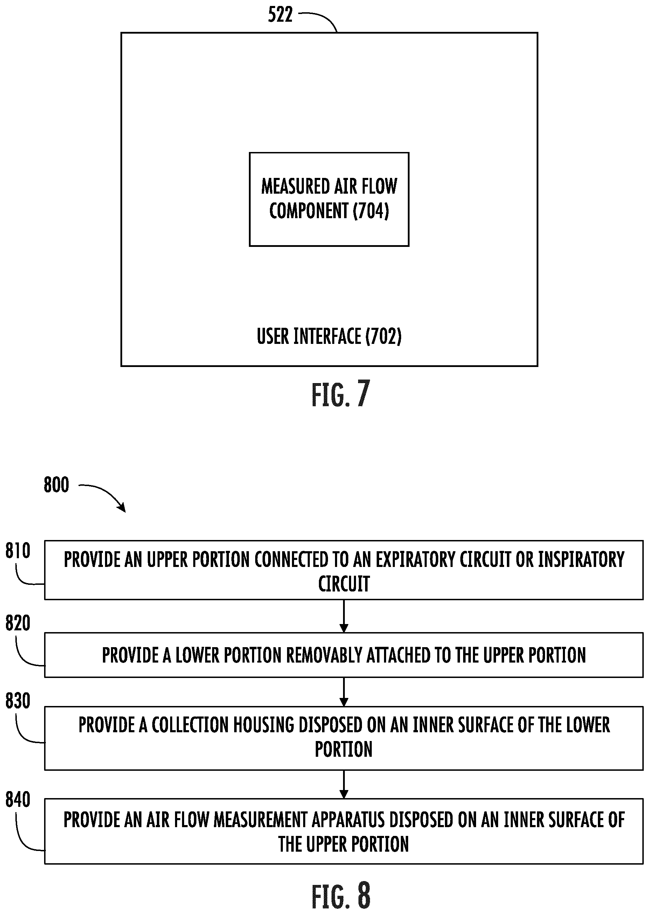

With reference to FIG. 7, in some embodiments, the mechanical ventilation device 522 may include a user interface 702. In some embodiments, the user interface 702 may include a measured air flow component 704. The measured air flow component 704 may be configured to display one or more measurements made by the air flow measurement apparatus 514 (e.g., the one or more measurements transmitted from the controller 516 and received by the mechanical ventilation device 522) and/or one or more properties associated with the air flow determined by the mechanical ventilation device 522 performing further analysis on the one or more measurements made by the air flow measurement apparatus 514. For example, the measured air flow component 704 may be configured to display the one or more properties associated with the air flow measured by the air flow measurement apparatus 514, including one or more of a velocity, a pressure, a humidity, a temperature, an oxygen concentration or a presence of oxygen, a carbon dioxide concentration or a presence of carbon dioxide, a nitric oxide concentration or a presence of nitric oxide, a methane concentration or a presence of methane, a volatile organic compound concentration (e.g., benzene, ethylene glycol, formaldehyde, acetone, methylene chloride, tetrachloroethylene, toluene, xylene, and/or 1,3-butadiene) or a presence of a volatile organic compound, a mass, and a volume associated with the air flow. In this regard, for example, the measured air flow component 704 may display text, symbols, graphs, and/or colors indicating one or more measurements made by the air flow measurement apparatus 514. For example, the measured air flow component 704 may display text indicating the velocity of the air flow.

Although described herein with respect to the mechanical ventilation device 522 having a user interface 702 with a measured air flow component 704 configured to display one or more measurements made by the air flow measurement apparatus 514, it would be understood by one skilled in the field to which this disclosure pertains that, in some embodiments, the controller 516 may include a user interface having a measured air flow component configured to display one or more measurements made by the air flow measurement apparatus 514.

### Example Method of Manufacturing a Sensing and Secretion Bypass Apparatus

Referring now to FIG. 8, a flowchart providing an example method 800, which is not part of the invention, of manufacturing a sensing and secretion bypass apparatus is illustrated.

As shown in block 810, the method of manufacturing may include providing an upper portion connected to an expiratory circuit or inspiratory circuit. As described above, the upper portion may be connected to an expiratory circuit or inspiratory circuit. The upper portion may receive an air flow from the expiratory circuit or inspiratory circuit. The expiratory circuit or inspiratory circuit may be associated with a mechanical ventilation device. In some embodiments, there may be secretions present in the air flow. Once the air flow has been received into the upper portion, the air flow may travel through the upper portion in direction x.

As shown in block 820, the method of manufacturing may include providing a lower portion removably attached to the upper portion. As described above, the lower portion may be removably attached to the upper portion of the sensing and secretion bypass apparatus. The upper portion and the lower portion may define a chamber when attached (e.g., when the upper portion and the lower portion are attached). The lower portion may be removably attached to the upper portion via any means capable of making the lower portion removably attached to the upper portion.

As shown in block 830, the method of manufacturing may include providing a collection housing disposed on an inner surface of the lower portion. As described above, the collection housing may be in fluid communication with the chamber when the upper portion and the lower portion are attached. The collection housing may be configured to collect secretions present in the air flow. In this regard, as the air flow travels through the upper portion in direction x, the secretions present in the air flow may slowly begin to sink due to the force of gravity. In some embodiments, when the lower portion is attached to the upper portion, the secretions may collect in the collection housing (e.g., as the secretions sink due to the force of gravity).

As shown in block 840, the method of manufacturing may include providing an air flow measurement apparatus disposed on an inner surface of the upper portion. As described above, the air flow measurement apparatus may be configured to measure one or more properties associated with the air flow. The one or more properties associated with the air flow may include one or more of a velocity, a pressure, a humidity, a temperature, an oxygen concentration or a presence of oxygen, a carbon dioxide concentration or a presence of carbon dioxide, a nitric oxide concentration or a presence of nitric oxide, a methane concentration or a presence of methane, a volatile organic compound concentration (e.g., benzene, ethylene glycol, formaldehyde, acetone, methylene chloride, tetrachloroethylene, toluene, xylene, and/or 1,3-butadiene) or a presence of a volatile organic compound, a mass, and a volume associated with the air flow. As described above, the air flow measurement apparatus may be one or more of a hot-wire anemometer, thermopile, and/or differential pressure sensor or transducer.

As described above, in some embodiments, the air flow measurement apparatus may be configured to measure the one or more properties associated with the air flow when the lower portion is attached to the upper portion. In some embodiments, the air flow measurement apparatus may be configured to measure the one or more properties associated with the air flow when the lower portion is detached from the upper portion.

As described above, the sensing and secretion bypass apparatus may include a controller. The controller may be configured to receive one or more measurements made by the air flow measurement apparatus from the air flow measurement apparatus. The controller may be in communication with a mechanical ventilation device, such as via a wired connection and/or a wireless connection (e.g., Bluetooth). The controller may be configured to transmit one or more measurements made by the air flow measurement apparatus to the mechanical ventilation device (e.g., the one or more properties associated with the air flow measured by the air flow measurement apparatus). Similarly, the mechanical ventilation device may be configured to receive the one or more measurements made by the air flow measurement apparatus. Although the controller and the mechanical ventilation device are depicted as separate components in FIG. 5, it would be understood by one skilled in the field to which this disclosure pertains that, in some embodiments, the controller and the mechanical ventilation device may be a single component. For example, in some embodiments, the controller may be embodied within the mechanical ventilation device.

As described above, in some embodiments, the mechanical ventilation device may include a user interface. In some embodiments, the user interface may include a measured air flow component. The measured air flow component may be configured to display one or more measurements made by the air flow measurement apparatus (e.g., the one or more measurements transmitted from the controller and received by the mechanical ventilation device). Additionally, as described above, although described herein with respect to the mechanical ventilation device having a user interface with a measured air flow component configured to display one or more measurements made by the air flow measurement apparatus, it would be understood by one skilled in the field to which this disclosure pertains that, in some embodiments, the controller may include a user interface having a measured air flow component configured to display one or more measurements made by the air flow measurement apparatus.

### Example Computer Processing Device

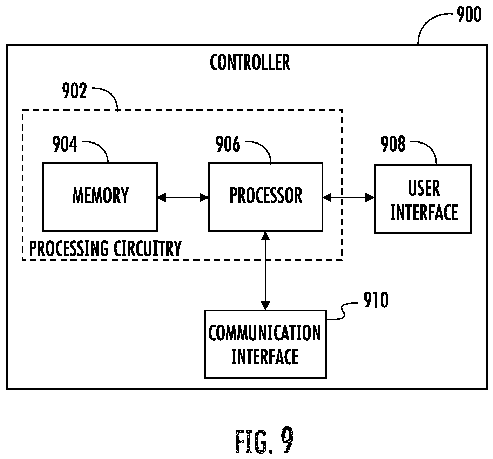

With reference to FIG. 9, a block diagram of an example computer processing device 900 is illustrated in accordance with some example embodiments. In some embodiments, the controller 104, the controller 516, and/or other devices (e.g., a controller associated with mechanical ventilation device 136 or mechanical ventilation device 522) may be embodied as one or more computer processing devices, such as the computer processing device 900 in FIG. 9. However, it should be noted that the components, devices, or elements illustrated in and described with respect to FIG. 9 below may not be mandatory and thus one or more may be omitted in certain embodiments. Additionally, some embodiments may include further or different components, devices or elements beyond those illustrated in and described with respect to FIG. 9.

The computer processing device 900 may include or otherwise be in communication with processing circuitry 902 that is configurable to perform actions in accordance with one or more embodiments disclosed herein. In this regard, the processing circuitry 902 may be configured to perform and/or control performance of one or more functionalities of the computer processing device 900 in accordance with various embodiments, and thus may provide means for performing functionalities of the computer processing device 900 in accordance with various embodiments. The processing circuitry 902 may be configured to perform data processing, application execution and/or other processing and management services according to one or more embodiments. In some embodiments, the computer processing device 900 or a portion(s) or component(s) thereof, such as the processing circuitry 902, may be embodied as or comprise a chip or chip set. In other words, the computer processing device 900 or the processing circuitry 902 may comprise one or more physical packages (e.g., chips) including materials, components and/or wires on a structural assembly (e.g., a baseboard). The structural assembly may provide physical strength, conservation of size, and/or limitation of electrical interaction for component circuitry included thereon. The computer processing device 900 or the processing circuitry 902 may therefore, in some cases, be configured to implement an embodiment of the disclosure on a single chip or as a single "system on a chip." As such, in some cases, a chip or chipset may constitute means for performing one or more operations for providing the functionalities described herein.

In some embodiments, the processing circuitry 902 may include a processor 906 and, in some embodiments, such as that illustrated in FIG. 9, may further include memory 904. The processing circuitry 902 may be in communication with or otherwise control a user interface 908 and/or a communication interface 910. As such, the processing circuitry 902 may be embodied as a circuit chip (e.g., an integrated circuit chip) configured (e.g., with hardware, software or a combination of hardware and software) to perform operations described herein.

The processor 906 may be embodied in a number of different ways. For example, the processor 906 may be embodied as various processing means such as one or more of a microprocessor or other processing element, a coprocessor, a controller or various other computing or processing devices including integrated circuits such as, for example, an ASIC (application specific integrated circuit), an FPGA (field programmable gate array), or the like. Although illustrated as a single processor, it will be appreciated that the processor 906 may comprise a plurality of processors. The plurality of processors may be in operative communication with each other and may be collectively configured to perform one or more functionalities of the computer processing device 900 as described herein. In some embodiments, the processor 906 may be configured to execute instructions stored in the memory 904 or otherwise accessible to the processor 906. As such, whether configured by hardware or by a combination of hardware and software, the processor 906 may represent an entity (e.g., physically embodied in circuitry - in the form of processing circuitry 902) capable of performing operations according to embodiments of the present disclosure while configured accordingly. Thus, for example, when the processor 906 is embodied as an ASIC, FPGA or the like, the processor 906 may be specifically configured hardware for conducting the operations described herein. Alternatively, as another example, when the processor 906 is embodied as an executor of software instructions, the instructions may specifically configure the processor 906 to perform one or more operations described herein.

In some embodiments, the memory 904 may include one or more non-transitory memory devices such as, for example, volatile and/or non-volatile memory that may be either fixed or removable. In this regard, the memory 904 may comprise a non-transitory computer-readable storage medium. It will be appreciated that while the memory 904 is illustrated as a single memory, the memory 904 may comprise a plurality of memories. The memory 904 may be configured to store information, data, applications, instructions and/or the like for enabling the computer processing device 900 to carry out various functions in accordance with one or more embodiments. For example, the memory 904 may be configured to buffer input data for processing by the processor 906. Additionally or alternatively, the memory 904 may be configured to store instructions for execution by the processor 906. As yet another alternative, the memory 904 may include one or more databases that may store a variety of files, contents or data sets. Among the contents of the memory 904, applications may be stored for execution by the processor 906 in order to carry out the functionality associated with each respective application. In some cases, the memory 904 may be in communication with one or more of the processor 906, user interface 908, and/or communication interface 910 via a bus(es) for passing information among components of the computer processing device 900.

The user interface 908 may be in communication with the processing circuitry 902 to receive an indication of a user input at the user interface 908 and/or to provide an audible, visual, mechanical or other output to the user. As such, the user interface 908 may include, for example, a keyboard, a mouse, a joystick, a display, a touch screen display, a microphone, a speaker, and/or other input/output mechanisms. As such, the user interface 908 may, in some embodiments, provide means for a user to access and interact with the controller 104, controller 516, mechanical ventilation device 136, and/or mechanical ventilation device 522.

The communication interface 910 may include one or more interface mechanisms for enabling communication with other devices and/or networks. In some cases, the communication interface 910 may be any means such as a device or circuitry embodied in either hardware, or a combination of hardware and software that is configured to receive and/or transmit data from/to a network and/or any other device or module in communication with the processing circuitry 902. By way of example, the communication interface 910 may be configured to enable the controller 104 to communicate with the air flow measurement apparatus 108, the mechanical ventilation device 136, and/or other controllers/computing devices. As another example, the communication interface 910 may be configured to enable the controller 516 to communicate with the air flow measurement apparatus 514, the mechanical ventilation device 522, and/or other controllers/computing devices. Accordingly, the communication interface 910 may, for example, include an antenna (or multiple antennas) and supporting hardware and/or software for enabling communications with a wireless communication network (e.g., a wireless local area network, cellular network, global positing system network, and/or the like) and/or a communication modem or other hardware/software for supporting communication via cable, digital subscriber line (DSL), universal serial bus (USB), Ethernet or other methods.

Many modifications and other embodiments of the inventions set forth herein will come to mind to one skilled in the art to which these inventions pertain having the benefit of teachings presented in the foregoing descriptions and the associated drawings. Although the figures only show certain components of the apparatus and systems described herein, it is understood that various other components may be used in conjunction with the system. Therefore, it is to be understood that the inventions are not to be limited to the specific embodiments disclosed and that modifications and other embodiments are intended to be included within the scope of the appended claims. Moreover, the steps in the method described above may not necessarily occur in the order depicted in the accompanying diagrams, and in some cases one or more of the steps depicted may occur substantially simultaneously, or additional steps may be involved. Although specific terms are employed herein, they are used in a generic and descriptive sense only and not for purposes of limitation.

While various embodiments in accordance with the principles disclosed herein have been shown and described above, modifications thereof may be made by one skilled in the art without departing from the teachings of the disclosure. The embodiments described herein are representative only and are not intended to be limiting. Many variations, combinations, and modifications are possible and are within the scope of the disclosure. Alternative embodiments that result from combining, integrating, and/or omitting features of the embodiment(s) are also within the scope of the disclosure. Accordingly, the scope of protection is limited by the claims.

Additionally, the section headings used herein are provided for consistency with the suggestions under 37 C.F.R. 1.77 or to otherwise provide organizational cues. These headings shall not limit or characterize the invention(s) set out in any claims that may issue from this disclosure.

Use of broader terms such as "comprises," "includes," and "having" should be understood to provide support for narrower terms such as "consisting of," "consisting essentially of," and "comprised substantially of" Use of the terms "optionally," "may," "might," "possibly," and the like with respect to any element of an embodiment means that the element is not required, or alternatively, the element is required, both alternatives being within the scope of the embodiment(s). Also, references to examples are merely provided for illustrative purposes, and are not intended to be exclusive.

## Claims

1. A sensing and secretion bypass apparatus (100), the sensing and secretion bypass apparatus (100) comprising:
an air flow sensor (102);
an adapter (110) comprising a chamber (112) having a first end (114) configured to receive an air flow and a second end (116) connected to the air flow sensor (102), wherein the air flow comprises secretions;
an elevated member (118) disposed within the chamber (112) proximate the second end (116) and configured to prevent the secretions from entering the air flow sensor (102); and
a disposal housing (132) in fluid communication with the chamber (112) of the adapter via a tube (126) connecting the disposal housing (132) to a bottom side (124) of the chamber (112) and configured to collect the secretions received via the first end of the chamber (112);
wherein the elevated member (118) is sloped between a first end (120) and a second end (122), wherein the elevated member (118) is disposed within the chamber (112) such that the first end (120) of the elevated member (118) is in contact with the bottom side (124) of the chamber (112) at a downstream edge of the tube (126), and the second end (122) of the elevated member (118) is separated from the bottom side (120) of the chamber (112) by a first distance less than a height of the chamber and is downstream of the first end.

2. The sensing and secretion bypass apparatus (100) of Claim 1, wherein the first end (114) is configured to receive the secretions from an expiratory circuit or inspiratory circuit (128).

3. The sensing and secretion bypass apparatus (100) of Claim 1, wherein the air flow sensor (102) is configured to receive the air flow without the secretions from the second end (116) of the chamber (112).

4. The sensing and secretion bypass apparatus (100) of Claim 1, wherein the air flow sensor (102) comprises an air flow measurement apparatus (108) configured to measure one or more properties associated with the air flow.

5. A method of manufacturing a sensing and secretion bypass apparatus (100), the method of manufacturing comprising:
providing an air flow sensor (102);
providing an adapter (110) comprising a chamber (112) having a first end (114) configured to receive an air flow and a second end (116) connected to the air flow sensor (102), wherein the air flow comprises secretions;
providing an elevated member (118) disposed within the chamber (112) proximate the second end (116) and configured to prevent the secretions from entering the air flow sensor (102); and
providing a disposal housing (132) in fluid communication with the chamber (112) of the adapter (110) via a tube (126) connecting the disposal housing (132) to a bottom side (124) of the chamber (112) and configured to collect the secretions received via the first end (114) of the chamber; wherein the
elevated member (118) is sloped between a first end (120) and a second end (122), wherein the elevated member (118) is disposed within the chamber (112) such that the first end (120) of the elevated member (118) is in contact with the bottom side (124) of the chamber (112) at a downstream edge of the tube (126), and the second end (122) of the elevated member (118) is separated from the bottom side (120) of the chamber (112) by a first distance less than a height of the chamber and is downstream of the first end.

## Patentansprüche

1. Erfassungs- und Sekretumgehungseinrichtung (100), die Erfassungs- und Sekretumgehungseinrichtung (100) umfassend:
einen Luftstromsensor (102),
ein Passstück (110), umfassend eine Kammer (112), die ein erstes Ende (114), das dazu konfiguriert ist, einen Luftstrom aufzunehmen, und ein zweites Ende (116), das mit dem Luftstromsensor (102) verbunden ist, aufweist, wobei der Luftstrom Sekrete umfasst;
ein aufgeständertes Element (118), das innerhalb der Kammer (112) nahe zu dem zweiten Ende (116) angeordnet und dazu konfiguriert ist, zu verhindern, dass die Sekrete in den Luftstromsensor (102) gelangen; und
ein Entsorgungsgehäuse (132) in fluidmäßiger Kommunikation mit der Kammer (112) des Passstücks über einen Schlauch (126), der das Entsorgungsgehäuse (132) mit einer Unterseite (124) der Kammer (112) verbindet und dazu konfiguriert ist, die über das erste Ende der Kammer (112) empfangenen Sekrete aufzufangen;
wobei das aufgeständerte Element (118) zwischen einem ersten Ende (120) und einem zweiten Ende (122) abgeschrägt ist, wobei das aufgeständerte Element (118) innerhalb der Kammer (112) so angeordnet ist, dass das erste Ende (120) des aufgeständerten Elements (118) in Kontakt mit der Unterseite (124) der Kammer (112) an einem nachgelagerten Rand des Schlauchs (126) ist, und das zweite Ende (122) des aufgeständerten Elements (118) durch einen ersten Abstand, der geringer als eine Höhe der Kammer ist, von der Unterseite (120) der Kammer (112) getrennt ist und von dem ersten Ende nachgelagert ist.

2. Erfassungs- und Sekretumgehungseinrichtung (100) nach Anspruch 1, wobei das erste Ende (114) dazu konfiguriert ist, die Sekrete aus einem Ausatmungskreislauf oder einem Einatmungskreislauf (128) zu empfangen.

3. Erfassungs- und Sekretumgehungseinrichtung (100) nach Anspruch 1, wobei der Luftstromsensor (102) dazu konfiguriert ist, den Luftstrom ohne die Sekrete von dem zweiten Ende (116) der Kammer (112) zu empfangen.

4. Erfassungs- und Sekretumgehungseinrichtung (100) nach Anspruch 1, wobei der Luftstromsensor (102) eine Luftstrommesseinrichtung (108) umfasst, die dazu konfiguriert ist, eine oder mehrere dem Luftstrom zugeordnete Eigenschaften zu messen.

5. Verfahren zum Herstellen einer Erfassungs- und Sekretumgehungseinrichtung (100), das Verfahren zum Herstellen umfassend:
Bereitstellen eines Luftstromsensors (102),
Bereitstellen eines Passstücks (110), umfassend eine Kammer (112), die ein erstes Ende (114), das dazu konfiguriert ist, einen Luftstrom aufzunehmen, und ein zweites Ende (116), das mit dem Luftstromsensor (102) verbunden ist, aufweist, wobei der Luftstrom Sekrete umfasst;
Bereitstellen eines aufgeständerten Elements (118), das innerhalb der Kammer (112) nahe zu dem zweiten Ende (116) angeordnet und dazu konfiguriert ist, zu verhindern, dass die Sekrete in den Luftstromsensor (102) gelangen; und
Bereitstellen eines Entsorgungsgehäuses (132) in fluidmäßiger Kommunikation mit der Kammer (112) des Passstücks (110) über einen Schlauch (126), der das Entsorgungsgehäuse (132) mit einer Unterseite (124) der Kammer (112) verbindet und dazu konfiguriert ist, die über das erste Ende (114) der Kammer empfangenen Sekrete aufzufangen;
wobei das aufgeständerte Element (118) zwischen einem ersten Ende (120) und einem zweiten Ende (122) abgeschrägt ist, wobei das aufgeständerte Element (118) innerhalb der Kammer (112) so angeordnet ist, dass das erste Ende (120) des aufgeständerten Elements (118) in Kontakt mit der Unterseite (124) der Kammer (112) an einem nachgelagerten Rand des Schlauchs (126) ist, und das zweite Ende (122) des aufgeständerten Elements (118) durch einen ersten Abstand, der geringer als eine Höhe der Kammer ist, von der Unterseite (120) der Kammer (112) getrennt ist und von dem ersten Ende nachgelagert ist.

## Revendications

1. Dispositif de détection et de dérivation de sécrétions (100), le dispositif de détection et de dérivation de sécrétions (100) comprenant :
un capteur de flux d'air (102) ;
un adaptateur (110) comprenant une chambre (112) présentant une première extrémité (114) configurée pour recevoir un flux d'air et une deuxième extrémité (116) reliée au capteur de flux d'air (102), dans lequel le flux d'air comprend des sécrétions ;
un élément surélevé (118) disposé à l'intérieur de la chambre (112) à proximité de la deuxième extrémité (116) et configuré pour empêcher les sécrétions de pénétrer dans le capteur de flux d'air (102) ; et
un réservoir de récupération (132) en communication fluidique avec la chambre (112) de l'adaptateur par l'intermédiaire d'un tube (126) reliant le réservoir de récupération (132) à une face inférieure (124) de la chambre (112) et configuré pour recueillir les sécrétions reçues par l'intermédiaire de la première extrémité de la chambre (112) ;
dans lequel l'élément surélevé (118) est incliné entre une première extrémité (120) et une deuxième extrémité (122), dans lequel l'élément surélevé (118) est disposé à l'intérieur de la chambre (112) de telle sorte que la première extrémité (120) de l'élément surélevé (118) est en contact avec la face inférieure (124) de la chambre (112) au niveau d'un bord en aval du tube (126), et la deuxième extrémité (122) de l'élément surélevé (118) est séparée de la face inférieure (120) de la chambre (112) par une première distance inférieure à une hauteur de la chambre et se situe en aval de la première extrémité.

2. Dispositif de détection et de dérivation de sécrétions (100) de la revendication 1, dans lequel la première extrémité (114) est configurée pour recevoir les sécrétions provenant d'un circuit expiratoire ou d'un circuit inspiratoire (128).

3. Dispositif de détection et de dérivation de sécrétions (100) de la revendication 1, dans lequel le capteur de flux d'air (102) est configuré pour recevoir le flux d'air sans les sécrétions depuis la deuxième extrémité (116) de la chambre (112).

4. Dispositif de détection et de dérivation de sécrétions (100) de la revendication 1, dans lequel le capteur de flux d'air (102) comprend un dispositif de mesure de flux d'air (108) configuré pour mesurer une ou plusieurs propriétés associées au flux d'air.

5. Procédé de fabrication d'un dispositif de détection et de dérivation de sécrétions (100), le procédé de fabrication comprenant :
la mise à disposition d'un capteur de flux d'air (102) ;
la mise à disposition d'un adaptateur (110) comprenant une chambre (112) présentant une première extrémité (114) configurée pour recevoir un flux d'air et une deuxième extrémité (116) reliée au capteur de flux d'air (102), dans lequel le flux d'air comprend des sécrétions ;
la mise à disposition d'un élément surélevé (118) disposé à l'intérieur de la chambre (112) à proximité de la deuxième extrémité (116) et configuré pour empêcher les sécrétions de pénétrer dans le capteur de flux d'air (102) ; et
la mise à disposition d'un réservoir de récupération (132) en communication fluidique avec la chambre (112) de l'adaptateur (110) par l'intermédiaire d'un tube (126) reliant le réservoir de récupération (132) à une face inférieure (124) de la chambre (112) et configuré pour recueillir les sécrétions reçues par l'intermédiaire de la première extrémité (114) de la chambre ;
dans lequel l'élément surélevé (118) est incliné entre une première extrémité (120) et une deuxième extrémité (122), dans lequel l'élément surélevé (118) est disposé à l'intérieur de la chambre (112) de telle sorte que la première extrémité (120) de l'élément surélevé (118) est en contact avec la face inférieure (124) de la chambre (112) au niveau d'un bord en aval du tube (126), et la deuxième extrémité (122) de l'élément surélevé (118) est séparée de la face inférieure (120) de la chambre (112) par une première distance inférieure à une hauteur de la chambre et se situe en aval de la première extrémité.
